# EUROPEAN PATENT SPECIFICATION

(11) **EP 1 635 843 B1**
(45) Date of publication and mention of the grant of the patent: **07.01.2009**
(21) Application number: 04744181.1
(22) Date of filing: 25.06.2004
(51) Int. Cl.: A61K 31/569, A61K 45/06, A61K 31/565, A61P 25/24

(54) **THERAPY COMPRISING DIENOGEST FOR HORMONE REPLACEMENT AND DEPRESSION**
THERAPIE FÜR HORMONERSATZ UND DEPRESSION ENTHALTEND DIENOGEST
HORMONOTHERAPIE SUBSTITUTIVE ET TRAITEMENT DE LA DEPRESSION COMPORTANT L'ADMINISTRATION D'UN DIENOGEST

(30) Priority: 25.06.2003 US 482154 P
(43) Date of publication of application: 22.03.2006
(73) Proprietor: Bayer Schering Pharma Aktiengesellschaft, 13353 Berlin (DE)
(72) Inventor: RUDOLPH, Ina, 41464 Neuss (DE); GRASER, Thomas, 99102 Windisch-Holzhausen (DE)
(86) International application number: PCT/IB2004/002535
(87) International publication number: WO 2004/112797

(56) References cited:
- WO-A-00/59577
- WO-A-03/041718
- WO-A-03/082299
- US-B1- 6 312 722
- GRÄSER ET AL: "Climodien (estradiol valerate 2 mg plus dienogest 2 mg) is safe and effective in the treatment of postmenopausal complaints" CLIMACTERIC, vol. 4, 2001, pages 332-342, XP008038644
- SALETU: "Veränderungen von Vigilanz, kognitiver Informationsverarbeitung und Schlafqualität unter Climodien" GYNE, 2001, pages 258-264, XP008038730
- SCHMIDT ET AL: "Estrogen replacement in perimenopause-related depression: a preliminary report" AM J OBSTET GYNECOL, vol. 183, no. 2, 2000, pages 414-420, XP008038729

## Description

Hormone replacement, especially estrogen replacement, therapy and contraception using hormone are well known in the art.

In climacteric women, anxiety, depression, tension and irritability begin during the perimenopause and can be correlated to reduced sexual hormone levels, for example, estrogen levels. Epidemiological studies show that approximately 22-33 % of climacteric women suffer from depression symptoms as levels of sexual hormones decrease. In about 10 % of these cases, symptoms become severe enough to indicate clinically relevant depression. Data suggests that especially estrogen, or the relative lack thereof, is especially implicated in the regulation of mood and behavior, as well as well as in the pathobiology of mood disorders during a woman's whole lifecycle.

According to US 6,077,852, there is a growing perception in the literature that estrogen replacement therapy is a promising treatment for central nervous system disorders such as depression and mood swings and of Alzheimer's disease in postmenopausal women. These promising uses of estrogen replacement therapy are offset, however, by the disadvantages of long-term estrogen therapy associated with the risks of developing reproductive tissue cancers.

Currently prescribed hormone replacement therapies (HRT), often utilize a progestin that is co-administered with estrogen to limit the estrogen's uterine stimulatory effects, thereby reducing the risk of endometrial cancer. Treatment with an estrogen alone is also possible. In most cases, women with a uterus indeed must be given estrogen and a progestin either together or more commonly in a cyclic protocol.

Co-administration of progestin has several undesirable side-effects, that are often poorly tolerated by many women, such as continuing menstrual periods, depression, edema, lower abdominal cramps, breast tenderness, and symptoms like premenstrual syndrome. The effects of the progestin can even negate the salutary effects of the estrogen. The estrogen, itself, also has several side effects, often causing, for example, water retention, weight gain, hypertension, etc. See, for example, US 6,326,366 and US 5,814329.

In the field of contraceptives, oral contraceptives are most prominent. Two types of agents are (a) estrogen combined with a progestin, and (b) a progestin alone. The contraceptives of the combined type act primarily by suppressing ovulation by negative feedback to prevent gonadotropin (LH and FSH) release by the hypothalamus, but alterations in the reproductive tract may also contribute to the antifertility effect. The action of a progestin alone in a very low oral dose ("mini-pill") appears to involve primarily alterations in the female reproductive tract, but ovulation suppression may also occur.

Although the oral contraceptives are highly effective, their use is also associated with unpleasant side effects, such as nausea, depression, weight gain, and headache, and an increased long-time risk of severe disease, such as thromboembolism, stroke, myocardial infarction, hepatic adenoma, gall bladder disease, and hypertension. Bleeding irregularities, such as break-through bleeding, spotting, and amenorrhea, are also frequent. A progestin, when administered alone, causes an increased incidence of changes in menstrual patterns, especially a marked increase in the amount and duration of menstrual bleeding. See for example, US 6,355,670.

In this invention the co-administration of an estrogen with the progestin dienogest (chemical name: 17-Hydroxyl-3-oxo-19-nor-17a-pregna-4,9-diene 21-nitrite, or 17alpha-Cyanomethyl-17β-hydroxy-4, 9-estradiene-3-one; chemical formula: C₂₀H₂₅NO₂) for HRT and/or contraception is useful in the treatment and/or prevention of depression.

With the use of an estrogen and dienogest, a patient can enjoy the usual benefits of contraception and hormone replacement therapy, for example, the treatment and/or prevention of irregular bleeding, hot flushes, sleep disturbances, mood swings, vaginal dryness, bladder problems, for example, incontinence, bone loss/osteoporosis, worsening of mind, memory and cognitive functions, e.g., verbal and non-verbal memory functions, worsening of vigilance, attention, and concentration, worsening of psychological well being and quality of life, skin and hair problems, and body shape changes, while simultaneously not only avoiding the side effect of depression often caused by the combination of estrogens and progestins, but if such depression was preexistent, the treatment thereof. Especially pronounced is the effect of estrogen and dienogest on preexistent depression in postmenopausal women. Positive effects on menopause specific (vasomotor and somatic symptoms, depressive mood, sexual behavior, memory / concentration problems, attractiveness) and mental health related quality of life (energy / vitality, social functioning, role limitations due to emotional problems, and mental health), were also observed after the administration of an estrogen with the new progestin dienogest.

Estrogens are well known in the art. Any estrogen is useful in the invention, for example, without limitation, estriol, estrone, estrone sulfate, estradiol-3, 17β-diproprionate, ethinylestradiol, 17β-estradiol as well as esters thereof, such as estradiol-3-benzoate, estradiol-17-valerate, -cyprionate, -undecylate, -enanthate and/or other esters (US-A-2,611,773, US-A-2,990,414, US-A-2,054,271, US-A-2,225,419 and US-A-2,156,599) and conjugated estrogens. Estradiol-, ethinylestradiol- and estrone-sulfamates, for example estrone-N,N-dimethylsulfamate, estrone-N,N-diethylsulfamate, ethinylestradiol-3-N,N-dimethylsulfamate, ethinylestradiol-3-N,N-diethylsulfamate, ethinylestradiol-3-N,N-tetramethylenesulfamate, estrone sulfamate, estradiol-3-sulfamate, estradiol-3-N,N-dimethylsulfamate, estradiol-3-N,N-diethylsulfamate, and ethinylestradiol-3-sulfamate, which produce all prodrugs of the corresponding 3-hydroxy compounds (W. Elger et al., in J. Steroid Biochem. Molec. Biol., Vol. 55, No. 3/4, 395-403, 1995, DE 44 29 398 Al and DE 44 29 397 A1), and 14α,17α-ethano-1,3,5(10)-estratriene-3,17β-diol, 14α,17α-ethano-1,3,5(10)estratiene-3, 16α,17β-triol or the 15,15-dialkyl derivatives of estradiol, as well as 14α,17α-methylene steroids from the estrane series and the corresponding 3-amidosulfonate derivatives can also be used according to the invention. Also useful are equilin, equilenin, dihydroequilenin, 17.beta.-dihydroequilenin, menstranol, equol or enterolactone, and sulfate esters thereof, for example, sodium estrone sulfate, sodium equilin sulfate, sodium 17alpha-dihydroequilin sulfate, sodium 17alpha-estradiol sulfate, sodium selta8,9-dehydroestrone sulfate, sodium equilenin sulfate, sodium 17beta-dihydroequilin sulfate, sodium 17alpha-dihydroequilenin sulfate, sodium 17beta-estradiol sulfate, sodium 17beta-dihydroequilenin sulfate, estrone 3-sodium sulfate, equilin 3-sodium sulfate, 17alpha-dihydroequilin 3-sodium sulfate, 3beta-hydroxy-estra-5(10), 7-dien-17-one 3-sodium sulfate, 5alpha-pregnan-3beta-20R-diol 20-sodium sulfate, 5alpha-pregnan-3beta, 16alpha-diol,20-one 3-sodium sulfate, delta(8,9)-dehydroestrone 3-sodium sulfate, estra-3beta, 17alpha-diol 3-sodium sulfate, 3beta-hydroxy-estr-5(10)-en, 17-one 3-sodium sulfate or 5alpha-pregnan-3beta,16alpha, 20R-triol 3-sodium sulfate. Preferred are estriol, estrone, estrone sulfate, estradiol-3, 17β-diproprionate, ethinylestradiol, 17β-estradiol as well as esters thereof, such as estradiol-3-benzoate, estradiol-17-valerate, -cyprionate, -undecylate, -enanthate and/or other esters (US-A-2,611,773, US-A-2,990,414, US-A-2,054,271, US-A-2,225,419 and US-A-2,156,599) and conjugated estrogens.

Doses and modes of administration for contraception and hormone replacement therapy are the customary modes and amounts administered in these fields for estrogens and progestins.

In hormone replacement therapy particularly suitable doses of estrogen and dienogest, each independently, can be 0.5 to 5 mg/day, preferably 1 to 4 mg/day, and especially preferably about 2 to 3 mg/day. In one especially preferred HRT embodiment, 2 mg/day of estrogen is co-administered with 2 mg/day of dienogest. In another preferred embodiment 2 mg/day of estrogen is co-administered with 3 mg/day of dienogest. The estrogen in these preferred embodiments is estradiol valerate, although it is not limited thereto. Of course, lower or higher doses can be used, especially in the case of conventional low dose estrogens like ethinyl estradiol, which is know to be used at dosages as low as, e.g., 10 µg, 30 µg, etc.

Suitable administration modes can be, without limitation, enteral, parenteral or oral administration, preferably oral administration. Administration can be, without limitation, sequential or simultaneous, e.g., combined in a single dosage form, preferably combined.

Dienogest does not produce the usual anti-estrogenic effect of other progestin in hormone replacement therapy and/or in its use as a contraceptive. Dienogest also lacks anti-estrogenic effects and androgenic effects, and has antiandrogenic effects. Dienogest thereby does not seem to counteract the positive effects of estrogen with regard to psychological functioning. Contrary to the general assumption about progestins, dienogest seems to increase instead of decrease the positive neurophysiological effects of estrogen, e.g., vigilance-promoting effects (Saletu, Veränderungen von vigilanz, kognitiver informationsverarbeitung und schlafqualität unter Climodien, Gyne Sept. 2001; Saletu et al.).

An advantage of the invention is the continuous combination of the estrogen with the progestin, suppressing cyclic change in sex hormones as one risk factor for mood alterations.

Preferred aspects herein disclosed are:
Treating and/or preventing depression in a woman undergoing contraception or hormone replacement therapy comprising administering to said woman a composition comprising an estrogen and dienogest;
Treating depression in a woman undergoing contraception or hormone replacement therapy, said woman having symptoms of depression, comprising administering to said woman a composition comprising an estrogen and dienogest;
Treating depression in a climacteric woman undergoing hormone replacement therapy comprising administering to said woman a composition comprising an estrogen and dienogest;
Treating depression in a climacteric woman undergoing hormone replacement therapy, said woman having symptoms of depression, comprising administering to said woman a composition comprising an estrogen and dienogest;
Treating depression in a postmenopausal woman undergoing hormone replacement therapy comprising administering to said woman a composition comprising an estrogen and dienogest;
Treating depression in a postmenopausal woman undergoing hormone replacement therapy, said woman having symptoms of depression, comprising administering to said woman a composition comprising an estrogen and dienogest;
Contraception and/or hormone replacement therapy in a woman, said woman having symptoms of depression, comprising administering to said woman a composition comprising an estrogen and dienogest;
Contraception and/or hormone replacement therapy in a woman, said woman having symptoms of depression, comprising administering to said woman a composition comprising dienogest;
Hormone replacement therapy in a woman, for example in a woman who is postmenopausal, said woman having symptoms of depression, wherein the woman optionally has been diagnosed with depression, for example, depression in the context of a postmenopausal syndrome, comprising administering to said woman a composition comprising an estrogen and dienogest;
Hormone replacement therapy in a woman, said woman having symptoms of depression or a depressive mood (not merely mood swings), comprising administering to said woman a composition comprising an estrogen and dienogest, or diegonest without an estrogen, wherein as a result of hormone replacement therapy, irregular bleeding, hot flushes, sleep disturbances, mood swings, vaginal dryness, bladder problems, bone loss/osteoporosis, worsening of cognitive functions or of vigilance, attention, or concentration, skin and hair problems, and body shape changes, are treated and/or are prevented and/or controlled;
Method wherein the woman undergoing hormone replacement therapy is a postmenopausal woman who has been diagnosed with depression, for example, depression in the context of a postmenopausal syndrome;
Treatment wherein the woman undergoing hormone replacement therapy and/or contraception or is being treated for depression has been diagnosed with depression according to an official classification system, such as, for example, the International Statistical Classification of Diseases and Related Health Problems, tenth revision (ICD-10) or Diagnostic and Statistical Manual of Mental Disorders - Fourth Edition (DSM-IV), not merely suffering from postmenopausal syndrome;
Treating and/or preventing a positive anamnesis for estrogen-dependent depressive disorder, for example, premenstrual syndrome (PMS) and/or postnatal depression (PND), in a woman in her fertile phase of life comprising administering to said woman a composition comprising an estrogen and dienogest;
Treating and/or preventing depression in a woman having PMS and/or PND comprising administering to said woman a composition comprising an estrogen and dienogest;
Treatment wherein the dose of estrogen and dienogest, each independently, is 0.5 to 5.0 mg/day;
Treatment wherein the dose of estrogen and dienogest, each, is about 2 mg/day; and
Treatment wherein the estrogen is estradiol valerate.

The disclosure also relates to:
Use of an estrogen and diegonest according to any of the preceding treatments for the production of pharmaceutical agents or medicaments;

Use of an estrogen and diegonest for the production of a pharmaceutical agent for:
treating and/or preventing depression or a depressive mood (not merely mood swings), in a woman undergoing contraception or hormone replacement therapy, which is the subject of the claimed invention; treating depression in a woman undergoing contraception or hormone replacement therapy, said woman having symptoms of depression; treating depression in a climacteric woman undergoing hormone replacement therapy comprising administering to said woman a composition comprising an estrogen and dienogest; treating depression in a climacteric woman undergoing hormone replacement therapy, said woman having symptoms of depression; treating depression in a postmenopausal woman undergoing hormone replacement therapy; treating depression in a postmenopausal woman undergoing hormone replacement therapy, said woman having symptoms of depression; contraception and/or hormone replacement therapy in a woman, said woman having symptoms of depression; contraception and/or hormone replacement therapy in a woman, said woman having symptoms of depression; hormone replacement therapy in a woman, for example in a woman who is postmenopausal, said woman having symptoms of depression, wherein the woman optionally has been diagnosed with depression, for example, depression in the context of a postmenopausal syndrome; hormone replacement therapy in a woman, said woman having symptoms of depression, for treating, preventing or controlling irregular bleeding, hot flushes, sleep disturbances, mood swings, vaginal dryness, bladder problems, bone loss/osteoporosis, worsening of cognitive functions or of vigilance, attention, or concentration, skin and hair problems, and body shape changes; hormone replacement therapy in a postmenopausal woman who has been diagnosed with depression, for example, depression in the context of a postmenopausal syndrome; hormone replacement therapy and/or contraception or treating for depression a woman who has been diagnosed with depression according to an official classification system, such as, for example, the International Statistical Classification of Diseases and Related Health Problems, tenth revision (ICD-10) or Diagnostic and Statistical Manual of Mental Disorders - Fourth Edition (DSM-IV), not merely suffering from postmenopausal syndrome; treating and/or preventing a positive anamnesis for estrogen-dependent depressive disorder, for example, premenstrual syndrome (PMS) and/or postnatal depression (PND), in a woman in her the fertile phase of life; treating and/or preventing depression in a woman having PMS and/or PND comprising administering to said woman a composition comprising an estrogen and dienogest;
Use according to any of the previous uses: where the dose of estrogen and dienogest, each independently, is 0.5 to 5.0 mg/day; wherein the dose of estrogen and dienogest, each, is about 2 mg/day; and wherein the estrogen is estradiol valerate;
Use of an estrogen and diegonest in the form of a pharmaceutical preparation for enteral, parenteral and oral administration; and
Use of estrogen and diegonest in the form of a preparation for enteral, parenteral and oral administration.

Upon further study of the specification and appended claims, further objects and advantages of this invention will become apparent to those skilled in the art.

Without further elaboration, it is believed that one skilled in the art can, using the preceding description, utilize the present invention to its fullest extent.

In the foregoing and in the following examples, all temperatures are set forth uncorrected in degrees Celsius and, all parts and percentages are by weight, unless otherwise indicated.

### EXAMPLES:

### Example 1:

The main objective of this multicenter, randomized, double-blind, placebo-controlled two-art trial over 24 weeks study was to investigate the effects of continuous-combined HRT with 2 mg Estradiol valerate (EV) and 2 mg Dienogest (DNG), product identified as Climodien, on psychological well-being, such as postmenopausal depression, in postmenopausal women. The women were diagnosed with postmenopausal depression according to the internationally accepted classification systems. They are not suffering merely from depressed mood and the beneficial effects established below were not shown merely from a few weeks, but much longer.

The primary efficacy variable was depression severity, as measured by the Hamilton Depression Rating Scale (HAMD) after 24 weeks of treatment. A 4-point difference between Climodien and Placebo at the end of the study was considered as clinically relevant.

One-hundred and twenty-nine patients with depression were selected according to: a) ICD-10:F32.0, F32.1 (mild to moderate depressive episbde), in the context of a postmenopausal syndrome (ICD10:N95.1); and b) baseline HAMD score of ≥ 16 . (ICD = International Classification of Diseases)

In the FAS (full analysis set) population, treatment with Climodien for 24 weeks led to a significant long-term reduction of the HAMD score (8.9 ± 6.4; p = 0.0167) compared to Placebo (12.8±8.5) (see below). In particular, the goal of 4 points difference between treatments, stated as clinically relevant difference, was achieved at final visit (about 9 absolute points for Climodien versus about 13 for Placebo, i.e., a difference of about 4 points). Also, 24-week treatment with Climodien achieved a mean reduction of 50% of the HAMD score (from 18.9 ± 3.1 to 8.9 ± 6.4). This was not achieved in the Placebo group (from 18.8± 3.9 to 12.8 ± 8.5). In clinical practice, a 50% reduction of the HAMD score is considered clinically relevant; also, a score of ≤ 8 points is considered a sign of remission of depression. Indeed, a sum score of ≤ 10 is considered characteristic of non-depressed patients.

### HAMD SCORE

| | | Climodien | | | Placebo | |
|---|---|---|---|---|---|---|
| | Mean ± SD | Lower CL | Upper CL | Mean ± SD | Lower CL | Upper CL |
| Baseline | 18.9±3.1 | - | - | 18.8±3.9 | - | - |
| | (n = 65) | | | (n = 64) | | |
| Week (Wk) 12 | 10.1±5.6 | - | - | 12.8 ± 7.4 | - | - |
| | (n = 51) | | | (n = 32) | | |
| Final (Week 24) | 8.9±6.4* | 7.1 | 10.7 | 12.8±8.5 | 100 | 15.6 |
| | (n=51) | | | (n = 38) | | |
| Mean differ. Wk 12 *minus* Baseline | -8.6±5.1° | -10.1 | -7.2 | -6.0±7.2° | -8.6 | -3.4 |
| | (n = 51) | | | (n = 32) | | |
| Mean differ. Final *minuis* Baseline | 9.7± 6.2° | -11.5 | -8.0 | -6.4 t 7.7° | -9.0 | -3.9 |
| | (n = 51) | | | (n=38) | | |

| | | | | | | |
|---|---|---|---|---|---|---|
| CL = Confidence limit * p = 0.0167 in the comparison between Climodien and Placebo at Week 24 (t test) ° p= < 0.0001 compared to baseline (within-group analyses) (t test) | | | | | | |

Notably, the changes within the Climodien group were significant both after 12 weeks (-8.6 points) and 24 weeks (-9.7 points) (p < 0.0001 compared to baseline); however there also was a strong, significant placebo effect at both time points (-6.0 points after 12 weeks and -6.4 points after 24 weeks) (p < 0.0001 compared to baseline).

In spite of their magnitude, the placebo effects at both 12 weeks and more significantly after 24 weeks were clearly exceeded by the effects of Climodien (p = 0.4167). The null hypothesis could then be rejected and the alternative hypothesis (superiority of Climodien) accepted. This data indicates that Climodien has both short term and long term effects on depression.

The effect of Climodien also seemed to be independent of the improvement in vasomotor symptoms and sleep disturbances, thus indicating a direct mood-enhancing effect. The data supported a partial dependence of the treatment effect on depression severity from the change in vasomotor symptoms and sleep disturbances, but there still remained a significant treatment effect of Climodien.

As evident from the n numbers of the table above, the Placebo group was characterized by several cases of drop-out (largely because of lack of efficacy). In order to estimate the influence of the differential drop-out rate in the two treatment groups, the analyses were repeated using the last-observation-carry-forward (LOCF) technique, a common method for the replacement of missing data. The significant differences between Climodien and Placebo at final visit were confirmed in this analysis (p = 0.0016).

### HAMD SCORE USING THE LOCF TECHNIQUE:

| | | Climodien (n=65) | | | Placebo (n=64) | |
|---|---|---|---|---|---|---|
| | Mean±SD | Lower CL | Upper CL | Mean ± SD | Lower CL | Upper CL |
| Baseline | 18.9±3.1 | - | - | 18.8±3.9 | - | - |
| "Weck (Wk) 12 | 12.1±6.4 | - | - | 15.8±6.9 | - | - |
| Final (Week 24) | 10.8±7.2* | 9.0 | 12.6 | 15.0±7.7 | 13.1 | 17.0 |
| Mean differ. Wk 12 *minus* Baseline | -6.8±5.7° | -8.2 | -5.3 | -3.0±5.9° | -4.5 | - |
| Mean differ. Final *minus* Baseline | -8.0±6.6° | -9.7 | -6.4 | -3.7±7.1° | -5.4 | -1.9 |

| | | | | | | |
|---|---|---|---|---|---|---|
| *p=0.0016 for the comparison between Climodien and Placebo at Week 24 (t test) ° p =<_ 0.0001 compared to baseline (within-group analyses) (t test) | | | | | | |

The t test data on depression severity (Climodien versus Placebo) was already significant after 12 weeks (t = -3.14, p = 0.002). Additionally, further significant decrease in depression severity was evident in only the Climodien treated group after 24 weeks as compared to 12 weeks (t = -2.16, p = 0.03).

The majority of women of the Climodien group responded well to treatment. The number of women with signs of remission of depression in the sense of a final score in the HAMD ≤ 8 points (corresponding to an improvement of ≥50%) in the Climodien treated group was very high. On the other hand, the majority of subjects treated with Placebo did not respond to treatment at all or prematurely discontinued treatment mostly for lack of efficacy.

| RESPONSE TO TREATMENT AS DESCRIBED BY HAMD FINAL SCORES | | |
|---|---|---|
| | Climodien® n= 65 (100%) | Placebo n= 64 (100%) |
| No response to treatment or dropout (HAMD ≥ 16) | 20 (30.8%) | 41 (64.1%) |
| Partial response (HAMD 9-15) | 17 (26.1%) | 6 (9.4%) |
| Complete response (HAMD ≤ 8) | 28 (43.1 %) | 17(26.6%) |

| | | |
|---|---|---|
| A final HAMD ≤ 8 corresponds to an improvement of > 50 %. | | |

The Clinical global impression CGI Resulsts were also consistent with these findings.

(CGI)/Severity of illness: At Week 6,12, and 24 (final visit), there was a clear shift from more severe scores to less severe ones. While a clear placebo effect was recognizable through the treatment periods, at Week 12 and 24 the frequency of patients with no or only mild symptomatology was clearly higher in the Climodien group.

CGI/Global improvement: While strong placebo effects were clearly recognizable through the study, these tended to diminish with time; in contrast, the effects observed in the Climodien group remained quite constant through time and were stronger than in the Placebo group.

CGI/Therapeutic effects: The positive effects of Climodien were very clear. The efficacy categories (marked, moderate, and minimal) clearly predominated in the Climodien group, whereas the unchanged or worsening categories clearly predominated in the Placebo group.

CGI/Side effects: Notably, unlike for the previous categories of the CGI, the category "side effects" displayed hardly any differences between the Climodien and the Placebo group, indicating a very good tolerability of Climodien.

The following table summarizes the therapeutic effect (efficacy index of CGI) results at final visit. The majority of women treated with Climodien® had moderate to marked improvement, i.e., complete or nearly complete remission of symptoms, the majority of women treated with Placebo had unchanged or worse results.

| THERAPEUTIC EFFECT AT THE END OF TREATMENT | | |
|---|---|---|
| | Climodien® n=60(100%) | Placebo n=57(100%) |
| Not assessed | 1 ( 1.7%) | 4 (7.0%) |
| Marked improvement | 21 (35.0%) | 7 (12.3%) |
| Moderate improvement | 19 (31.7%) | 12 (21.1%) |
| Minimal improvement | 7 (11.7%) | 5 ( 8.8%) |
| Unchanged or worse | 12 (20.0%) | 29 (50.9%) |

The above described results, i.e., the effects of Climodien on depression, were also well supported by data on menopause-specific Women's Health Questionnaire (WHQ) and mental health related Short Form Health Survey (SF-36) Quality of Life (QoL) asessment.

In sum, the continuous-combined HRT with Climodien for 24 weeks induced a significant improvement of postmenopausal depression compared to Placebo (p = 0.0167), in spite of a strong placebo effect.

### Example 2:

The main objective of this study was the assessment of the dependence of the effect of treatment on the intensity of depression (HAMD) on a positive anamnesis for estrogen-dependent depressive disorders (PMS and/or PND) in the fertile phase of life.

Within the context of this clinical study, the presence of the premenstrual syndrome (PMS) and postnatal depression (PND) was included in the patients' files, examinations by the physicians, etc. The anamnesis was then considered positive if at least one of the disorders could be detected from it. PND occurred by anamnesis only together with PMS.

The review of a dependency of the effect of Climodien^{®} on the depression intensity of a positive anamnesis for PMS and/or PND was carried out by means of a simple analysis of variance (ANOVA) with repeated measurements. The model included the factors of PMS and/or PND (present vs. absent) and time (baseline, 12 and 24 weeks). The depression intensity (HAMD) was a dependent variable.

A significant difference between women with or without PMS/PND in depression intensity (HAMD) in the baseline was observed. Women with PMS/PND were significantly more depressed in this case. With treatment, a significant improvement in the depression intensity resulted in both groups. Even women without PMS/PND seemed to respond well to treatment with Climodien^{®}.

| DESCRIPTIVE STATISTICS FOR THE DEPRESSION INTENSITY (HAMD CUMULATIVE SCORE) FOR WOMEN WITH AND WITHOUT PMS/PND. | | |
|---|---|---|
| | With PMS/PND (n=17) Mean ± SD | Without PMS/PND (n = 48) Mean ± SD |
| Baseline | 21.7 ±2.7 | 17.9 ±2.5 |
| Week 12 | 14.5 ± 7.9 | 11.2±5.6 |
| Final (Week 24) | 13.4±8.7 | 9.9±6.5 |
| Mean Difference | -7.2 ± 7.1 | -6.6 ± 5.3 |
| Week 12 without Baseline | | |
| Mean Difference | -8.4 ± 7.7 | -7.9 ± 6.3 |
| Final minus Baseline | | |

The entire disclosure[s] of all applications, patents and publications, cited herein are mentioned herein, including US 60/482,154, filed on June 25, 2003, and US 60/488,439, filed on July 21, 2003.

The preceding example can be repeated with similar success by substituting the generically or specifically described reactants and/or operating conditions of this invention for those used in the preceding examples.

## Claims

1. Use of an estrogen and dienogest for the production of a pharmaceutical agent for treating and/or preventing depression or a depressive mood in a woman undergoing contraception or hormone replacement therapy.

2. Use of a daily dose of estrogen of 10 µg to 5.0 mg and of a daily dose of 0.5 to 5.0 mg of dienogest according to claim 1.

3. Use of 2 mg estrogen and 2 mg Dienogest according to claim 2.

4. Use according to claim 2 or 3 wherein the estrogen is estradiol valerate.

5. Use according to claim 1, 2, 3 or 4 wherein the estrogen and dienogest is in the form of a pharmaceutical preparation for enteral, parenteral or oral administration.

## Patentansprüche

1. Verwendung eines Östrogens und Dienogest zur Herstellung eines pharmazeutischen Mittels zur Behandlung und/oder Prävention von Depression oder einer depressiven Stimmung bei einer Frau, die sich einer Empfängnisverhütung oder einer Hormonersatztherapie unterzieht.

2. Verwendung einer Östrogen-Tagesdosis von 10 µg bis 5,0 mg und einer Dienogest-Tagesdosis von 0,5 bis 5,0 mg nach Anspruch 1.

3. Verwendung von 2 mg Östrogen und 2 mg Dienogest nach Anspruch 2.

4. Verwendung nach Anspruch 2 oder 3, wobei es sich bei dem Östrogen um Östradiolvalerat handelt.

5. Verwendung nach einem der Ansprüche 1 bis 4, wobei das Östrogen und Dienogest in Form einer pharmazeutischen Zubereitung zur enteralen, parenteralen oder oralen Verabreichung vorliegt.

## Revendications

1. Utilisation d'un estrogène et du diénogest pour la production d'un agent pharmaceutique destiné au traitement et/ou à la prévention de la dépression ou d'une humeur dépressive chez une femme suivant une contraception ou un traitement hormonal substitutif.

2. Utilisation d'une dose journalière d'estrogène de 10 µg à 5,0 mg et d'une dose journalière de 0,5 à 5,0 mg de diénogest selon la revendication 1.

3. Utilisation de 2 mg d'estrogène et de 2 mg de diénogest selon la revendication 2.

4. Utilisation selon la revendication 2 ou 3, **caractérisée en ce que** l'estrogène est le valérate d'estradiol.

5. Utilisation selon la revendication 1, 2, 3 ou 4, **caractérisée en ce que** l'estrogène et le diénogest sont sous forme d'une préparation pharmaceutique destinée à l'administration par voie entérale, parentérale ou orale.
